# EUROPÄISCHE PATENTSCHRIFT

(11) **EP 0 497 796 B1**
(45) Veröffentlichungstag und Bekanntmachung des Hinweises auf die Patenterteilung: **22.06.1994**
(21) Anmeldenummer: 90915126.8
(22) Anmeldetag: 18.10.1990
(51) Int. Cl.: C07B 37/06, C07C 25/13, C07C 205/12

(54) **VERFAHREN ZUR HERSTELLUNG VON FLUORBENZOLEN**
METHOD OF PREPARATION OF FLUOROBENZENES
PROCEDE DE FABRICATION DE FLUOROBENZENES

(30) Priorität: 27.10.1989 DE 3935862
(43) Veröffentlichungstag der Anmeldung: 12.08.1992
(73) Patentinhaber: HOECHST AKTIENGESELLSCHAFT, 65926 Frankfurt am Main (DE)
(72) Erfinder: SISTIG, Frank, Peter, D-6238 Hofheim am Taunus (DE); LEUPOLD, Ernst, Ingo, D-6392 Neu-Anspach (DE); LITTERER, Heinz, D-6208 Bad Schwalbach (DE)
(86) Internationale Anmeldenummer: EP9001762
(87) Internationale Veröffentlichungsnummer: WO9106518

(56) Entgegenhaltungen:
- DE-A- 3 824 141
- Collection of Czechoslovak Chemical Communications, Band 37, 1972, Prag, CH; J. SMOLIK et al.:"Decarbonylation of aromatic aldehydes on platinum metal catalysts", S. 3042-3051

## Beschreibung

Die vorliegende Erfindung betrifft ein Verfahren zur Herstellung von gegebenenfalls substituierten Fluorbenzolen durch thermische Decarbonylierung der entsprechenden Fluorbenzaldehyde mit Hilfe von Übergangsmetall-Katalysatoren.Viele der substituierten Fluorbenzole bilden wertvolle Zwischenstufen bei der Herstellung von Verbindungen mit herbizider, fungizider oder insektizider Wirkung; sie sind ebenso einsetzbar für die Herstellung von wichtigen pharmazeutischen Wirksubstanzen.

Bisher wurden Fluorbenzole aus den entsprechenden substituierten Anilinen durch Diazotierung und anschließenden Ersatz der Diazo-Gruppe durch Fluor hergestellt. So ist die Herstellung von Fluorbenzol durch Diazotierung von Anilin-hydrochlorid, Umwandlung des erhaltenen Benzoldiazonium-chlorids in das Tetrafluoroborat und nachfolgendes Erhitzen seit langem bekannt (G. Balz und G. Schiemann, Ber. 60 (1927) 1188; D.T. Flood, Org. Synth. Coll. Vol. II (1943) 295). Daneben ist die Herstellung von Fluorbenzol auch durch Diazotierung von Anilin in wasserfreiem Fluorwasserstoff bei 0°C mit nachfolgender Zersetzung des erhaltenen Benzoldiazonium-fluorids bei 20°C beschrieben (Kirk-Othmer, Encyclopedia of Chemical Technology, 3rd ed., Vol. 10, S. 908). 1,3-Difluor-benzol konnte analog durch Erhitzen von Benzol-1,3-bis-diazoniumtetrafluoroborat in 31 %iger Ausbeute erhalten werden, bezogen auf m-Phenylendiamin als Ausgangsverbindung (G. Schiemann und R. Pillarsky, Ber. 62 (1929) 3035-3043, speziell 3029). Die Diazotierung von 3-Fluor-anilin in wasserfreiem Fluorwasserstoff in Gegenwart von entweder Ammoniumfluorid oder tertiären Aminen oder Dimethylsulfoxid ergab gleichfalls 1,3-Difluor-benzol in einer Ausbeute von 46 bis 73 % (US-PS 4 075 252 und US-PS 4 096 196).

Die Decarbonylierung aromatischer Aldehyde unter Ersatz der Formyl-Gruppe durch Wasserstoff ist eine in der Literatur vielfach beschriebene Reaktion. Sie wird katalysiert unter anderem durch Übergangsmetalle wie Chrom, Mangan, Nickel, Kupfer oder Zink, insbesondere aber durch die Metalle der Platin-Gruppe. Aus Kostengründen werden diese Metalle in der Regel auf inerten Trägermaterialien niedergeschlagen. Es ist jedoch auch die Verwendung löslicher Edelmetallkomplexe beschrieben, wodurch eine Reaktionsführung in homogener Lösung möglich wird (Houben-Weyl, Methoden der Organischen Chemie, Bd. V/2b (1981) 332-336, Georg Thieme Verlag, Stuttgart).

Die Decarbonylierung fluorsubstituierter Benzaldehyde gelang bisher jedoch nur in einer ökonomisch unvertretbar geringen Ausbeute. So wurde 1,3-Difluor-benzol durch Erhitzen von 2,6-Difluor-benzaldehyd in 50 %iger wäßriger Kalilauge in einer Ausbeute von etwa 70 % erhalten (G. Lock, Ber. 69 (1936) 2253).

Nach dem Balz-Schiemann-Verfahren können unsubstituierte oder substituierte Fluorbenzole häufig nur in ungenügender Ausbeute erhalten werden. Es ist darüberhinaus generell mit dem verfahrenstechnischen Nachteil verbunden, daß große Mengen an Salz anfallen. Es bestand daher die Aufgabe, ein Verfahren zu entwickeln, nach dem in hoher Ausbeute Fluorbenzole, die noch weiter substituiert sein können, aus den entsprechenden Fluorbenzaldehyden erhalten werden können.

Es wurde nun gefunden, daß Fluorbenzole in hoher Ausbeute durch thermische Decarbonylierung aus fluorsubstituierten Benzaldehyden erhalten werden können, wenn die gebildeten Fluorbenzole unmittelbar aus der Reaktionszone entfernt werden. Da die Fluorbenzole einen niedrigeren Siedepunkt besitzen als die Fluorbenzaldehyde, geschieht dies besonders einfach, indem man Bedingungen anwendet, unter denen das gewünschte Fluorbenzol flüchtig ist und abgetrennt werden kann.

Gegenstand der Erfindung ist somit ein Verfahren zur Herstellung von Fluorbenzolen mit mindestens einem Wasserstoffatom als Kernsubstituenten und gegebenenfalls weiteren Substituenten,die unabhängig voneinander Chlor, Brom, Nitro, Hydroxy, C₁-C₃-Alkoxy oder C₁-C₃-Alkyl sein können, wobei die Anzahl der Nitrogruppen nicht mehr als 2 und die der Hydroxy- und Alkoxygruppen jeweils nicht mehr als 3 beträgt, durch Erhitzen der entsprechenden mit mindestens einem Fluoratom substituierten Benzaldehyd in Gegenwart eines Katalysators, dadurch gekennzeichnet, daß das Reaktionsprodukt unmittelbar aus der Reaktionszone abgetrennt wird. Zweckmäßig beträgt dabei die Anzahl der Nitro- und Hydroxygruppen jeweils nicht mehr als 2 und die der Hydroxy- und Alkoxygruppen zusammen nicht mehr als 3.

Geeignete Ausgangsmaterialien für das erfindungsgemäße Verfahren sind Benzaldehyde, die mit einem oder mehreren Fluoratomen substituiert sind; daneben kann noch mindestens ein anderer Substituent vorhanden sein, beispielsweise Chlor, Brom, Nitro, Hydroxy, C₁-C₃-Alkoxy und/oder C₁-C₃-Alkyl.

Geeignete Katalysatoren für das erfindungsgemäße Verfahren enthalten zweckmäßig ein oder mehrere Übergangsmetalle der I., II., VI., VII. und VIII. Nebengruppe, wie Chrom, Mangan, Nickel, Kupfer oder Zink, bevorzugt jedoch ein oder mehrere Metalle aus der Gruppe der Platin-Metalle, insbesondere Rhodium. Dabei kann im heterogenen System mit festen, auf Trägern befindlichen Katalysatoren oder im homogenen System in flüssiger Phase gearbeitet werden.

Lösliche Rhodium-Komplexe, mit denen in einem homogenen flüssigen System gearbeitet werden kann oder mit denen Träger imprägniert werden können sind beispielsweise Rhodium(I)-Komplexe wie ClRh(PPh₃)₃ ("Wilkinson-Katalysator"), ClRh(CO)(PPh₃)₂, [ClRh(CO)₂]₂, acacRh(CO)(PPh₃), acacRh(CO)₂, (C₅H₅)Rh(C₈H₁₄) und (C₃H₅)Rh(PPh₃)₂, wobei Ph für Phenyl, acac für Acetylacetonat, C₈H₁₄ für Cycloocten, C₅H₅ für Cyclopentadienyl und C₃H₅ für Allyl stehen. Geeignet sind auch Rhodium(II)- und Rhodium(III)-Komplexe wie Rhodium(II)-acetat, Rhodium(II)-2,4-difluor-benzoat, Rh(acac)₃, RhCl₃·xH₂O, Rh(NO₃)₃ und (C₃H₅)RhCl₂(PPh₃)₂. Zu diesen Rhodium-Komplexen können vorteilhaft noch Verbindungen, die als Liganden wirken können, wie Phosphane, Phosphite oder Amine, zugesetzt werden.

Das erfindungsgemäße Verfahren kann in allen für Flüssigphasen-Reaktionen geeigneten Apparaturen durchgeführt werden. Eine apparativ einfache Lösung ist ein Rührkessel mit aufgesetzter Destillationskolonne. Arbeitet man in einem heterogenen System, so kann der Katalysator in Suspension oder fest angeordnet vorliegen.

Druck und Temperatur werden im homogen wie im heterogen katalysierten Decarbonylierungsverfahren vorteilhaft so gewählt, daß eine möglichst vollständige Entfernung der gewünschten Reaktionsprodukte aus dem Reaktionsgemisch gewährleistet ist. Zweckmäßig arbeitet man bei einer Temperatur von 120 bis 300°C, bevorzugt 150 bis 200°C. Je nach Art des gewünschten Reaktionsproduktes kann bei einem Druck von 0,01 bis 10 bar gearbeitet werden. Bevorzugt liegt der Druck im Bereich von 0,1 bis 3 bar, besonders bevorzugt im Bereich von 1,0 bis 1,2 bar.

Das Ausgangsmaterial kann vollständig vorgelegt oder, was bevorzugt ist, laufend der Reaktionszone zugeführt werden. Auch eine Kombination ist möglich, also Vorlegen eines Teils und Nachdosieren des übrigen Teils. Bei Verwendung von solchen Ausgangsmaterialien, die den Katalysator allmählich desaktivieren, hat es sich als vorteilhaft erwiesen, kontinuierlich oder diskontinuierlich kleine Mengen des Reaktionsgemisches zu entnehmen und die Menge des entnommenen Katalysators durch eine äquivalente Menge an frischem Katalysator zu ersetzen.

Nach einer besonderen Ausführungsform des erfindungsgemäßen Verfahrens läßt man auf das Reaktionsgemisch Mikrowellen einwirken, wodurch eine nochmals höhere Ausbeute erzielt werden kann.

Die folgenden Vergleichsbeispiele V1 und V2 zeigen, daß der CO-Partialdruck (V1 1 bar, V2 25 bar) praktisch keinen Einfluß auf die Decarbonylierungsreaktion hat. Bei beiden Versuchen erhielt man nach einer Reaktionszeit von 72 h ca. 12 % 1,3-Difluor-benzol. Dies ist insofern bemerkenswert, als eine Erhöhung des CO-Partialdruckes in der Nähe des Reaktionsgleichgewichtes zu einer Verminderung der Ausbeute führen sollte. Die Decarbonylierung verläuft also nicht in der Nähe des Gleichgewichtes, und die entstandenen Reaktionsprodukte CO und das gegebenenfalls substituierte Fluorbenzol bewirken keine signifikante Verschiebung des Gleichgewichtes. Deshalb war es um so überraschender und nicht vorhersehbar, daß die unmittelbare Entfernung der Produkte aus dem Reaktionsgemisch zu hohen Umsätzen und Ausbeuten führt.

### Vergleichsbeispiele

V1. 258 g 2,4-Difluor-benzaldehyd und 3,3 g ClRh(PPh₃)₃ wurden in einem 500 ml Kolben mit Rückflußkühler bei 170°C unter einem CO-Druck von einer Atmosphäre gerührt. Nach 72 h wurde abgekühlt. Die Reaktionsmischung enthielt 31 g 1,3-Difluor-benzol (12,5 Gew.-%).
V2. 256 g 2,4-Difluor-benzaldehyd und 3,3 g ClRh(PPh₃)₃ wurden in einem 10 l Edelstahlautoklaven unter 22 bar CO-Druck bei 170°C gerührt. Der Druck stieg langsam auf 28 bar an. Nach 72 h wurde abgekühlt und die Reaktionsmischung analysiert. Sie enthielt 12,4 Gew.-% 1,3-Difluor-benzol (30,8 g).

### Beispiele

1. In einem 10 l Kolben mit Destillationsaufsatz, bestehend aus einer 50 cm langen Vigreux-Kolonne, Dephlegmator (betrieben bei 86°C) und Destillationskopf, wurde ein Rhodiumkatalysator mit 74 mmol Rh - hergestellt aus 20 g RhCl₃·xH₂O (38 % Rh-Gehalt), 50 g Triphenylphosphan, 15 g Wasser und 32 g 2,4-Difluorbenzaldehyd - in 9645 g 2,4-Difluor-benzaldehyd vorgelegt. Nach Aufheizen auf etwa 170°C und ca. 20 h Induktionszeit wurden je Stunde 21 g 1,3-Difluor-benzol gebildet. Mit sinkendem Flüssigkeitsstand nahm die Aktivität des Katalysators ab und nach 40 Tagen fiel kein Produkt mehr an. Die gesammelten Fraktionen von 7360 g mit Gehalten von über 99 % an 1,3-Difluor-benzol entsprachen einer Ausbeute von 95 %.
2. In einem 2 l Kolben mit automatischem Ausgleich des Flüssigkeitsstandes im Sumpf und dem in Beispiel 1 beschriebenen Destillationsaufsatz wurde ein Rhodium-Katalysator mit 40 mmol Rhodium zusammen mit 1500 g 2,4-Difluor-benzaldehyd vorgelegt und auf 168-175°C aufgeheizt. Je Stunde fielen in der Vorlage 20 bis 25 g 1,3-Difluor-benzol an, wobei umgesetzter 2,4-Difluorbenzaldehyd in der Apparatur laufend ersetzt wurde. Nach 1000 h Betriebszeit waren 19,62 kg 1,3-Difluor-benzol gewonnen. Die Analyse des Sumpfes ergab einen Rückstand von 3 Gew.-%, bezogen auf die eingesetzte Menge.
   Nach 1000 Betriebsstunden wurden 20 % des Sumpfes entnommen und die Menge des darin enthaltenen Katalysators durch eine äquivalente Menge an frischem Katalysator ersetzt, um eine kontinuierliche Betriebsweise zu ermöglichen.
3. In einer modifizierten Apparatur mit einem 1 l Reaktionskolben wurden 5,14 g RhCl₃·xH₂O (wie Beispiel 1), 15,7 g Triphenylphosphan und 495 g 2,4-Difluor-benzaldehyd vorgelegt, weitere 500 g 2,4-Difluor-benzaldehyd hinzugefügt und der Kolben dann mit Mikrowellen der Frequenz 2450 MHz und einer Leistung von ca. 350 W bestrahlt. Nach einer Induktionszeit von ca. 20 Stunden wurden täglich 278 g 1,3-Difluor-benzol (Gehalt über 99 %) gewonnen. 2,4-Difluor-benzaldehyd wurde kontinuierlich nachgefüllt, so daß der Füllstand im Reaktionskolben praktisch konstant blieb. Innerhalb von 100 h war keine Abnahme der Aktivität des Katalysators feststellbar.
4. In einem 500 ml Kolben mit Destillationsaufsatz wurden 40 g 2-Chlor-6-fluor-benzaldehyd und ein gemäß Beispiel 1 hergestellter Rhodiumkatalysator, der 1 mmol Rhodium enthielt, bei 200°C gerührt. Nach 400 h fiel kein Produkt mehr an, d.h. die Reaktion war beendet (Ausbeute 96 %).
5. Wie in Beispiel 4 beschrieben wurden 42 g 2-Fluor-5-nitro-benzaldehyd bei 215°C umgesetzt. Nach 200 h war die Reaktion beendet (Ausbeute 84 %).

## Patentansprüche

1. Verfahren zur Herstellung von Fluorbenzolen mit mindestens einem Wasserstoffatom als Kernsubstituenten und gegebenenfalls weiteren Substituenten, die unabhängig voneinander Chlor, Brom, Nitro, Hydroxy, C₁-C₃-Alkoxy oder C₁-C₃-Alkyl sein können, wobei die Anzahl der Nitrogruppen nicht mehr als 2 und die der Hydroxy- und Alkoxygruppen jeweils nicht mehr als 3 beträgt, durch Erhitzen der entsprechenden mit mindestens einem Fluoratom substituierten Benzaldehyde in Gegenwart eines Katalysators, dadurch gekennzeichnet, daß das Reaktionsprodukt unmittelbar aus der Reaktionszone in der der Benzaldehyd flüssig vorliegt abgetrennt wird.

2. Verfahren nach Anspruch 1, dadurch gekennzeichnet, daß der Katalysator mindestens ein Metall der I., II., VI., VII. und VIII. Nebengruppe, bevorzugt der VIII. Nebengruppe des Periodischen Systems, besonders bevorzugt Rhodium enthält.

3. Verfahren nach Anspruch 1 oder 2, dadurch gekennzeichnet, daß der Katalysator in homogener flüssiger Phase angewendet wird.

4. Verfahren nach einem oder mehreren der Ansprüche 1 bis 3, dadurch gekennzeichnet, daß man bei Temperaturen von 120 bis 300°C, bevorzugt 150 bis 200°C, arbeitet.

5. Verfahren nach einem oder mehreren der Ansprüche 1 bis 4, dadurch gekennzeichnet, daß der Reaktionsmischung Energie in Form von Mikrowellen zugeführt wird.

6. Verfahren nach einem oder mehreren dr Ansprüche 1 bis 5, dadurch gekennzeichnet, daß man Bedingungen anwendet, unter denen das gebildete Fluorbenzol flüchtig ist.

7. Verfahren nach einem oder mehreren der Ansprüche 1 bis 6, dadurch gekennzeichnet, daß 2,4-Difluor-benzaldehyd als Ausgangsmaterial eingesetzt wird.

## Claims

1. Process for the preparation of fluorobenzenes having at least one hydrogen atom as ring substituent and, optionally, further substituents, which, independently of each other, can be chlorine, bromine, nitro, hydroxyl, C₁-C₃-alkoxy or C₁-C₃-alkyl, the number of nitro groups being not more than 2 and the number of hydroxyl groups and alkoxy groups being not more than 3 in each case, by heating the corresponding benzaldehydes, substituted by at least one fluorine atom, in the presence of a catalyst, characterized in that the reaction product is immediately removed from the reaction zone in which the benzaldehyde is present as a liquid.

2. Process according to claim 1, characterized in that the catalyst contains at least one metal from subgroup I, II, VI, VII and VIII, preferably subgroup VIII, of the Periodic Table, particularly preferably rhodium.

3. Process according to claim 1 or 2, characterized in that the catalyst is employed in a homogeneous liquid phase.

4. Process according to one or more of claims 1 to 3, characterized in that temperatures of 120 to 300°C, preferably 150 to 200°C, are employed.

5. Process according to one or more of claims 1 to 4, characterized in that energy is supplied to the reaction mixture in the form of microwaves.

6. Process according to one or more of claims 1 to 5, characterized in that conditions are employed under which the fluorobenzene formed is volatile.

7. Process according to one or more of claims 1 to 6, characterized in that 2,4-difluorobenzaldehyde is employed as the starting material.

## Revendications

1. Procédé pour préparer des fluorobenzènes avec au moins un atome d'hydrogène comme substituant du noyau et éventuellement d'autres substituants, qui, indépendamment les uns des autres, peuvent être le chlore, le brome, le nitro, l'hydroxy, un alcoxy en C₁-C₃ ou un alkyle en C₁-C₃, le nombre des groupes nitro n'étant pas supérieur à 2 et les groupes hydroxy et alcoxy n'étant chaque fois pas supérieur à 3, par chauffage des benzaldéhydes correspondants substitués avec au moins un atome de fluor, en présence d'un catalyseur, le procédé étant caractérisé en ce que le produit de réaction est séparé immédiatement de la zone de réaction dans laquelle le benzaldéhyde se présente à l'état liquide.

2. Procédé selon la revendication 1, caractérisé en ce que le catalyseur contient au moins un métal des sous-groupes I., II., VI., VII., et VIII., de préférence le sous-groupe VIII., de la classification périodique, de façon particulièrement préférentielle le rhodium.

3. Procédé selon la revendication 1 ou 2, caractérisé en ce que le catalyseur est utilisé dans une phase liquide homogène.

4. Procédé selon une ou plusieurs des revendications 1 à 3, caractérisé en ce que l'on travaille à des températures de 120 à 300° C, de préférence à des températures de 150 à 200° C.

5. Procédé selon une ou plusieurs des revendications 1 à 4, caractérisé en ce que l'énergie est apportée au mélange réactionnel sous forme de micro-ondes.

6. Procédé selon une ou plusieurs des revendications 1 à 5, caractérisé en ce que l'on utilise des conditions dans lesquelles le fluorobenzène formé est volatil.

7. Procédé selon une ou plusieurs des revendications 1 à 6, caractérisé en ce que l'on utilise comme matière de départ le 2,4-difluorobenzaldéhyde.
